# EUROPÄISCHE PATENTSCHRIFT

(11) **EP 1 353 890 B1**
(45) Veröffentlichungstag und Bekanntmachung des Hinweises auf die Patenterteilung: **28.03.2007**
(21) Anmeldenummer: 02719713.6
(22) Anmeldetag: 23.01.2002
(51) Int. Cl.: C07C 15/28, C07C 7/14, C07D 209/84

(54) **GEWINNUNG VON ANTHRACEN UND CARBAZOL DURCH SCHMELZKRISTALLISATION**
OBTAINING ANTHRACENE AND CARBAZOLE BY MELT-CRYSTALLIZATION
EXTRACTION D'ANTHRACENE ET DE CARBAZOLE PAR CRISTALLISATION DE FUSION

(30) Priorität: 24.01.2001 DE 10103208
(43) Veröffentlichungstag der Anmeldung: 22.10.2003
(73) Patentinhaber: RÜTGERS Chemicals AG, 44579 Castrop-Rauxel (DE)
(72) Erfinder: BÜTTNER, Thomas, W., 53343 Wachtberg-Villip (DE); KNIPS, Ulrich, 59174 Kamen (DE); STOLZENBERG, Konrad, 45731 Waltrop (DE); TALBIERSKY, Jörg, 46282 Dorsten (DE); FUHRMANN, Edgar, 44579 Castrop-Rauxel (DE); ALSMEIER, Friedhelm, 45133 Essen (DE); BERGINS, Wolfgang, 44579 Castrop-Rauxel (DE); GIERTLER, Siegfried, 45768 Marl (DE); SCHOLL, Dietmar, 45470 Mühlheim/Ruhr (DE); VIERHAUS, Bernd, 58239 Schwerte (DE); DIALER, Klaus, CH-8409 Winterhur (CH); BISCHOF, Rudolf, CH-8880 Walenstadt (CH); NIKZAD, Ali, CH-8049 Zürich (CH)
(74) Vertreter: Minderop, Ralph H.
(86) Internationale Anmeldenummer: PCT/EP2002/000605
(87) Internationale Veröffentlichungsnummer: WO 2002/064533

(56) Entgegenhaltungen:
- DE-C- 19 613 497
- GERD COLLIN: "Ullmann's Encyclopedia of Industrial Chemistry - Anthracene" [Online] 15. Juni 2000 (2000-06-15), WILEY-VCH VERLAG GMBH & CO. KGAA Gefunden im Internet: URL:http://www.mrw.interscience.wiley.com/ ueic/articles/a02_343/sect1-fs.html DOI:10.1002/14356007.a02_343> [gefunden am 2004-08-17]

## Beschreibung

Die Erfindung betrifft ein Verfahren zur Gewinnung von Carbazol und Anthracen durch Schmelzkristallisation.

Carbazol ist ein Zwischenprodukt zur Herstellung von Farbstoffen, Pigmenten, Pestiziden und Polymeren. Die bedeutendsten Anwendungen für Carbazol sind Violett 23 und Hydronblau R. Das Pigment Violett 23 zeichnet sich durch hohe Farbstärke und Lichtechtheit aus. Es ist Basis für die Herstellung von Druckfarben und Autolacken sowie für die Einfärbung von Kunststoffen wie Polyvinylchlorid. Hydronblau R ist ein bedeutender lichtechter Farbstoff, der sich ausgezeichnet zum Färben von Baumwollstoffen eignet. Anthracen ist nach Oxidation zum Anthrachinon Ausgangsmaterial zur Herstellung von Anthrachinonfarbstoffen, deren Echtheitseigenschaften als sehr gut bezeichnet werden. Seit dem Beginn der Farbstoffchemie bilden sie neben den Azofarbstoffen die wichigste Farbstoffklasse.

Die Gewinnung von Anthrachinon und Carbazol aus dem Anthracenöl des Steinkohlenteers durch Kristallisation und Destillation in der Gasphase ist in der DE 196 13 497 C1 beschrieben und basiert auf einer Verwendung von Lösungsmitteln im Bereich von 120 bis 210°C. Diese Verfahren sind wegen des hohen Energiebedarfs zur Redestillation der Lösungsmittel sehr teuer.

Die in der DE 19 633 609 A1 beschriebene katalytische Synthese von Carbazol kann mit der Gewinnung von Carbazol aus Steinkohlenteer wirtschaftlich nicht konkurrieren. Die in der DE 19 757 530 A1 beschriebene Gewinnung von Anthracen und Carbazol auf der Basis der Hydrierung von Rohanthracen aus Steinkohlenteer ist aus Kostengründen derzeit nicht konkurrenzfähig. Die EP 799 813 beschreibt die benzofuranfreie Gewinnung von Anthracen oder von Anthrachinon durch Rektifikation.

GERD COLLIN: "Ullmann's Encyclopedia of Industrial Chemistry - Anthracene" [Online] 15. Juni 2000 (2000-06-15), WILEY-VCH VERLAG GMBH & CO. KGAA beschreibt die Gewinnung von Anthracen und Carbazol ausgehend von Anthracenöl durch Schmelzkristallisation ohne Zugabe von Lösungsmitteln. Anthracenöl, das bei der kontinuierlichen Kohledestillation in der Fraktion mit einem Siedepunkt zwischen 300 und 360 °C angereichert wird, wird auf 20 bis 30 °C abgekühlt und anschließend zentrifugiert. Hierbei wird Rohanthracenöl als gelb-grünes kristallines Material erhalten. Dieser Rückstand kann anschließend destilliert werden, wobei ein Rohprodukt erhalten wird, das 45 bis 55 % Anthracen enthält. Dieses Rohanthracen wird anschließend über Lösungsmittelkristallisation und über Destillation gereinigt.

Die der Erfindung zugrunde liegende Aufgabe besteht darin, ausgehend von Steinkohlenteerprodukten, wie Rohanthracen und Anthracenöl, benzofuranfreies Reinanthracen und ein farblich einwandfreies Carbazol bei erhöhter Raum-Zeit-Ausbeute und verbesserter Energieeffizienz bereitzustellen.

Verfahren zur Gewinnung von Anthracen und Carbazol und deren Folgeprodukten aus dem bei der Steinkohlenteerdestillation anfallenden Anthracenöl oder dessen Aufreinigungsprodukten, dadurch gekennzeichnet, daß man das Anthracenöl oder das Aufreinigungsprodukt Rohanthracen ohne Zugabe von Lösungsmittel in eine Schmelze überführt, die Schmelze unter den Kristallisationspunkt von Carbazol und Anthracen kühlt, das erhaltene Kristallgut von der flüssigen Phase trennt und zum Erhalt von Reinanthracen und Reincarbazol das Kristallgut destilliert, wodurch man die Schmelzkristallisation mehrstufig ausführt.
Mit dem erfindungsgemäßen Verfahren werden Anthracen und Carbazol bei hoher Reinheit ohne den Einsatz von Lösungsmitteln und ohne die üblicherweise im Anthracenöl vorhandenen Leichtsieder erhalten.

Ausgangsmaterial für das erfindungsgemäße Verfahren ist Rohanthracen oder Anthracenöl. Rohanthracen wird aus dem bei der Steinkohlenteerdestillation anfallenden Anthracenöl gewonnen. Die Steinkohlenteerdestillation und die Gewinnung von Rohanthracen sind beispielsweise beschrieben in Franck/Collin, Steinkohlenteer, Springer-Verlag 1968. Rohanthracen kann in bekannter Weise durch eine Kühlkristallisation, beispielsweise in einem Rührkristaller und anschließendem Zentrifugieren aus Anthracenöl gewonnen werden. Auf diese Art und Weise wird das sogenannte Rohanthracen 30 erhalten. Gemäß einer besonderen Ausführungform der Erfindung wird Rohanthracen durch statische Schmelzkristallisation gewonnen. Ausgangsmaterial des erfindungsgemäßen Verfahrens kann aber auch Anthracenöl sein. Einem solchen Anthracenöl können die leichtsiedenden Öle bei der vorangegangen Teerdestillation entzogen worden sein.

Das Anthracenöl wird in einem Kristaller, vorzugsweise einem statischen Kristaller, langsam auf eine Temperatur von 100 bis 20 °C abgekühlt. Dabei kristallisieren Anthracen, Carbazol und Phenanthren auf den Oberlächen des Kristallers. Nach Abkühlung der Schmelze auf 60 bis 20 °C wird das nicht erstarrte restliche Öl aus dem Kristaller abgezogen. Anschließend wird die an den Kristalleroberflächen befindliche Kristallschicht langsam erwärmt. Bei diesem Aufheizen des Kristallguts werden in den Kristallen enthaltene Verunreinigugen, Zwickelflüssigkeit und Fremdmoleküle, aufgeschmolzen und tropfen von der restlichen Kristallschicht ab. Dieser, als Schwitzen bezeichnete Vorgang wird so lange betrieben, bis eine genügend große Menge des Kristallguts, beispielsweise 1 bis 20 Gew.%, abgetropft sind. Die abgeschwitzte Ölmenge kann beispielswise durch die Niveauerfassung im Sumpf des Kristallers erfolgen.

Überraschenderweise gelingt dieser Reinigungsschritt auch ohne die üblicherweise zugesetzten Lösungsmittel und auch ohne die in der Siedelage von 280 bis 300 °C liegenden Öle wie Fluoren und Acenaphten, die das vor allem bei der Herstellung von Anthrachinon aus Anthracen störende Dibenzofuran enthalten.

Nach Abzug des Schwitzöls wird das restliche Kristallgut vollständig aufgeschmolzen und gesammelt werden. Dieses Öl entspricht in seiner Zusammensetzung dem herkömmlich gewonnenen Rohanthracen.

Dieses Material kann in weiteren Kristallisationsstufen in der zuvor beschriebenen Weise aufgereinigt werden. Je nach gewünschter Produktqualität wird die zuvor beschriebene Schmelzkristallisation mit dem Anschmelzen noch zwei bis vier Mal durchgeführt. Dabei wird Phenanthren von den ein Mischkristall bildenden Zielprodukten Anthracen und Carbazol getrennt. Vorzugsweise werden in den weiteren Kristallisationsstufen die Kristallisations- und die Schmelztemperaturen gegenüber den Temperaturen der vorherigen Stufe gesteigert.

Vorteilhaft ist, daß das erfindungsgemäße Verfahren mit einer großen Spanne von Anthracenölen oder Rohanthracen gefahren werden kann. Die eingesetzten Fraktionen können Konzentrationen von Anthracen in einem Bereich von 5 bis 40 Gew.%, von Carbazol in einem Bereich von 3 bis 25 Gew.% und eine Phenanthrenkonzentration von bis zu 35 Gew.% aufweisen. Als Produkt wird ein Anthracen-Carbazolgemisch mit Konzentrationen aller Begleitstoffe von 5 bis 1% in Summe erhalten.

Das erhaltene Kristallgut wird in bekannter Weise einer Destillation unterzogen, wobei Reinanthracen und Reincarbazol voneinander getrennt werden. Eine solche Destillation ist, allerdings ausgehend von Rohanthracen, beispielsweise in der DE 196 13 497 C1 beschrieben. Die Destillation des Gemischs aus Anthracen, Carbazol und einem geringen Anteil Phenanthren erfolgt vorzugsweise zur Vermeidung von Verpichungen und Verfärbungen und zur Minimierung des Energieverbrauchs unter Vakuum, besonders bevorzugt in einer Packungskolonne. Die Destillationskolonne weist mindestens etwa 60 theoretische Böden auf. An einer oberen Seitenabnahme der Kolonne wird die Reinanthracen, an einer unteren Seitenabnahme der Kolonne die Reincarbazol enthaltende Fraktion entnommen. Die Anthracenfraktion siedet bei einer Temperatur von 335 bis 345°C (Normaldruck), die Carbazolfraktion bei 345 bis 370°C (Normaldruck).

Zur Erhöhung der Reinheit des Anthracens kann die heißflüssige Anthracenfraktion in einer nachgeschalteten Kristallisation umkristallisiert werden. Dabei werden letzte Reste des siedebegleitenden Phenanthrens abgetrennt und die Reinheit des Anthracens auf 96 bis 99,5% und mehr erhöht. Die das Phenanthren enthaltende Fraktion aus diesem Prozeß kann zur Erhöhung der Ausbeute in die Kristallisation zurückgeführt werden.

Das erfindungsgemäße Verfahren ermöglicht auch den Einsatz eines Anthracenöls mit einem Siedebeginn von mehr als 300 °C als Ausgangsmaterial. Die nach der Schmelzkristallisation noch enthaltenden Reste an Dibenzofuran können bei der sich anschließenden Destillation zur Trennung des Anthracen-Carbazolgemischs als Kopfprodukt abgetrennt werden.

Die Oxidation von Anthracen zu Anthrachinon kann in bekannter Weise beispielsweise durch Luftsauerstoff und Wasserstoffperoxid erfolgen. Weitere Verfahren wie die Gasphasenoxidation sind beschrieben in H.-G. Franck, J.W. Stadelhofer, Industrielle Aromatenchemie, Springer-Verlag (1987), Seiten 358-360.

Die folgende beispielhafte Ausführungsform dient der weiteren Erläuterung der Erfindung.

Ein Anthracenöl mit einem Gehalt von 7% Anthracen, 25,1% Phenantren und 3,3% Carbazol wird in einem Aufheizer auf eine Temperatur von etwa 200 °C gebracht. Aus dem Aufheizer wird die erhaltene Schmelze auf einen Plattenkristaller gepumpt. Sie weisen zunächst eine Temperatur von ebenfalls etwa 200°C auf und werden dann gekühlt. Auf den Platten kristallisiert die Schmelze. Die Platten werden anschließend geringfügig derart aufgeheizt, daß ein Teil der auskristallisierten Schmelze wieder flüssig wird und abläuft. Die ablaufende Schmelze macht etwa 10 Gew.% des auf den Platten auskristallisierten Materials aus. Der abgeschwitzte Anteil wird aufgefangen und kann in die Rohanthracengewinnung rückgeführt werden.

Das auf den Platten verbliebene Material wird weiter aufgeheizt, zur Schmelze verflüssigt und zur weiteren Kristallisation auf die Platten eines zweiten Kristallers gepumpt. Die Platten weisen zunächst wieder eine Temperatur von oberhalb der Schmelztemperatur des Anhracen-Carbazolgemischs auf und werden dann gekühlt. Die Schmelze kristallisiert aus und erneut wird ein Anteil von etwa 10% des auskristallisierten Materials abgeschwitzt. Das dieser zweiten Kristallisationsstufe abgeschwitzte Material kann in die erste Kristallisationsstufe rückgeführt werden.

Das nach dem Abschwitzen in der zweiten Kristallisationsstufe erhaltene Material wird wieder aufgeschmolzen und über einen dritten Kristaller gegeben. Das Material wird auskristallisiert und einem erneuten Abschwitzen unterzogen. Das abgeschwitzte Material kann in die zweite Kristallisationsstufe rückgeführt werden.

Das verbleibende Material enthält etwa 63% Anthracen, 33% Carbazol und 2% Phenanthren.

Im Vergleich zu den üblicherweise eingesetzten Lösungsmittelkristallisationen auf deutlich geringerem Temperaturniveau (50-150°C), werden bei der erfindungsgemäßen Hochtemperatur-Schmelzkristallisation etwa gleiche Energiemengen für Heiz- und Kühlprozesse benötigt. Durch den Verzicht auf Lösungsmittel wird die zu heizende und kühlende Masse derart verringert, daß die höheren Temperaturen energetisch nicht relevant sind. In der folgenden Destillation zur Trennung von Anthracen und Carbazol verringert sich der Energiebedarf durch den Verzicht auf Lösungsmittel aber derart, daß der Gesamtenergiebedarf des Verfahrens unter 50% des Energiebedarfs der herkömmlichen Suspensionskristallisation sinkt.

## Patentansprüche

1. Verfahren zur Gewinnung von Anthracen und Carbazol und deren Folgeprodukten aus dem bei der Steinkohlenteerdestillation anfallenden Anthracenöl oder dessen Aufreinigungsprodukten, **dadurch gekennzeichnet, daß** man das Anthracenöl oder das Aufreinigungsprodukt Rohanthracen ohne Zugabe von Lösungsmittel in eine Schmelze überführt, die Schmelze unter den Kristallisationspunkt von Carbazol und Anthracen kühlt, das erhaltene Kristallgut von der flüssigen Phase trennt und zum Erhalt von Reinanthracen und Reincarbazol das Kristallgut destilliert, **dadurch gekennzeichnet, daß** man die Schmelzkristallisation mehrstufig ausführt.

2. Verfahren nach Anspruch 1, **dadurch gekennzeichnet, daß** man die Schmelzkristallisation zweistufig oder dreistufig ausführt.

3. Verfahren nach Anspruch 1 oder 2, **dadurch gekennzeichnet, daß** man die Schmelzkristallisation an einem Plattenkristaller durchführt.

4. Verfahren nach einem der Ansprüche 1 bis 3, **dadurch gekennzeichnet, daß** man etwa 55 bis 65 Gew.% der Schmelze an den Platten auskristallisieren läßt und die restliche Schmelze abtrennt.

5. Verfahren nach Anspruch 3 oder 4, **dadurch gekennzeichnet, daß** man das Kristallgut an den Platten schwitzen läßt und etwa 10% des zuvor an den Platten erstarrten Materials von denselben ablaufen läßt und die Platten erneut kühlt.

6. Verfahren nach einem der Ansprüche 1 bis 5, **dadurch gekennzeichnet, daß** man als Ausgangsmaterial ein Anthracenöl mit einem Siedebeginn bei einer Temperatur von mehr als 300 °C einsetzt.

7. Verfahren nach Anspruch 6, **dadurch gekennzeichnet, daß** man restliches Dibenzofuran als Kopfprodukt bei der destillativen Trennung des Anthracen-Carbazolgemischs abtrennt.

8. Verfahren nach Anspruch 1 oder 7, **dadurch gekennzeichnet, daß** man die durch Destillation gewonnene Anthracenfraktion umkristallisiert.

9. Verfahren nach einem der Ansprüche 1 bis 8, **dadurch gekennzeichnet, daß** man das erhaltene Reinanthracen zu Anthrachinon oxidiert.

10. Verfahren nach einem der Ansprüche 1 bis 9, **dadurch gekennzeichnet, daß** man das erhaltene Reincarbazol, Reinanthracen oder Anthrachinon granuliert, gegebenenfalls noch einmal mahlt, und verpackt.

## Claims

1. A method for preparing anthracene and carbazole and their sequential products from the anthracene oil resulting during coal tar distillation, or its purification products, **characterized in that** anthracene oil or its purification product crude anthracene is converted into a melt without adding solvent, the melt is cooled under the crystallization point of carbazole and anthracene, the crystal product obtained is separated from the liquid phase, and the crystal product is distilled to obtain pure anthracene and pure carbazole, **characterized in that** the method of claim 1, **characterized in that** the melt crystallization is performed in multiple stages.

2. The method of claim 1, **characterized in that** the melt crystallization is performed in two stages or in three stages.

3. The method of claim 1 or 2, **characterized in that** the melt crystallization is performed on a plate crystallizer.

4. The method of any of claims 1 to 3, **characterized in that** in approximately 55 to 65 weight-percent of the melt is allowed to crystallize out on the plates and the residual melt is separated off.

5. The method of claim 3 or 4, **characterized in that** the crystal product is caused to sweat on the plates and approximately 10% of the material previously solidified on the plates is allowed to drain off and the plates are cooled again.

6. The method of claim 5, **characterized in that** an anthracene oil with an initial boiling point at a temperature of more than 300°C is used as a starting material.

7. The method of claim 6, **characterized in that** the residual dibenzofuran is separated as an overhead product during the separation of the anthracene-carbazole mixture by distillation.

8. The method of any of claims 1 to 7, **characterized in that** the anthracene fraction obtained by distillation is recrystallized.

9. The method of any of claims 1 to 8, **characterized in that** the pure anthracene obtained is oxidized to anthraquinone.

10. The method of any of claims 1 to 9 **characterized in that** the pure carbazole, pure anthracene or pure anthraquinone is granulated, milled again, and packed.

## Revendications

1. Procédé d'obtention de l'anthracène et du carbazole et de leurs dérivés des huiles anthracéniques provenant de la distillation du goudron de houille ou de ses produits de purification, **caractérisé en ce que** l'on convertit les huiles anthracéniques ou le produit de purification, l'anthracène brut, en une masse fondue sans addition de solvant, la masse fondue est refroidie sous le point de cristallisation du carbazole et de l'anthracène, le produit de cristallisation obtenu est séparé de la phase liquide et pour obtenir l'anthracène pur ou le carbazole pur, on distille le produit de cristallisation, **caractérisé en ce que** l'on pratique la cristallisation de la masse fondue en plusieurs étapes.

2. Procédé selon la revendication 1, **caractérisé en ce que** l'on pratique la cristallisation de la masse fondue en deux étapes ou en trois étapes.

3. Procédé selon la revendication 1 ou 2, **caractérisé en ce que** l'on réalise la cristallisation de la masse fondue dans un cristalliseur à plaques.

4. Procédé selon l'une des revendications 1 à 3, **caractérisé en ce que** l'on fait recristalliser environ 55 à 65% en poids de la masse fondue sur les plaques et l'on sépare le reste de la masse fondue.

5. Procédé selon la revendication 3 ou 4, **caractérisé en ce que** l'on fait resuer le produit cristallin sur les plaques et que l'on fait écouler environ 10% du matériau d'abord pris sur les plaques et on refroidit à nouveau les plaques.

6. Procédé selon l'une des revendications 1 à 5, **caractérisé en ce que** l'on met en oeuvre comme matériau de départ, des huiles anthracéniques avec un commencement d'ébullition à une température de plus de 300°C.

7. Procédé selon la revendication 6, **caractérisé en ce que** l'on sépare le dibenzofuranne restant comme produit de tête dans la séparation par distillation du mélange anthracène-carbazole.

8. Procédé selon la revendication 1 ou 7, **caractérisé en ce que** l'on recristallise la fraction anthracène obtenue par distillation.

9. Procédé selon l'une des revendications 1 à 8, **caractérisé en ce que** l'on oxyde l'anthracène pur obtenu en anthraquinone.

10. Procédé selon l'une des revendications 1 à 9, **caractérisé en ce que** l'on granule le carbazole pur, l'anthracène pur ou l'anthraquinone obtenus, le cas échéant, on broie et on emballe.
